(19)

Europäisches
Patentamt
European
Patent Office
Office européen
des brevets

(11)  **EP 2 306 186 A2**

(12)  **EUROPEAN PATENT APPLICATION**

(43) Date of publication:
**06.04.2011 Bulletin 2011/14**

(51) Int Cl.:
**G01N 29/44** *(2006.01)*   **G01N 29/06** *(2006.01)*
**A61B 8/13** *(2006.01)*

(21) Application number: **10180938.2**

(22) Date of filing: **28.09.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **30.09.2009 JP 2009227226**

(71) Applicant: **Fujifilm Corporation
Minato-ku
Tokyo 106-8620 (JP)**

(72) Inventor: **Katsuyama, Kimito
Kanagawa 258-8538 (JP)**

(74) Representative: **Klunker . Schmitt-Nilson . Hirsch
Patentanwälte
Destouchesstrasse 68
80796 München (DE)**

(54)  **Ultrasound diagnostic apparatus and signal processing method thereof**

(57)     A region-of-interest and grid point setting unit is configured to set a region of interest (ROI) within an object and grid points to be measured within the ROI in the depth direction. An intra-region-of-interest sound velocity calculation unit is configured to calculate ambient sound velocities at the grid points at respective depths of the ROI. A local sound velocity determination unit is configured to compare the values of an ambient sound-velocity distribution table for each plurality of assumed local sound velocities with the values of the ambient sound velocities calculated by the intra-region-of-interest sound velocity calculation unit, extract an ambient sound-velocity distribution table for assumed local sound velocities having the smallest error, and determine the assumed local sound velocities of the extracted ambient sound-velocity distribution table as local sound velocities in the ROI.

EP 2 306 186 A2

**Description**

BACKGROUND OF THE INVENTION

Field of the Invention

The presently disclosed subject matter relates to an ultrasound diagnostic apparatus and the signal processing method thereof, and more particularly, to an ultrasound diagnostic apparatus and the signal processing method thereof for taking and displaying ultrasound images of an object by using ultrasound waves.

Description of the Related Art

Conventionally, attempts have been made to measure a sound velocity value (hereinafter referred to as a local sound velocity value) in a portion inside an object (site under diagnosis) by using ultrasound waves. For example, a method has been proposed in which two transmitting and receiving transducers are disposed oppositely to each other and a sound velocity value within the object is determined from a distance between the transducers and the propagation time of ultrasound waves. Another method has been proposed in which two pairs of transducers disposed at a predetermined distance from each other are respectively used for transmission and reception, and a propagation velocity is determined from the propagation time and the wave-transmitting and receiving angles of ultrasound waves between transducers and a distance between the respective pairs of transducers.

In addition, Japanese Patent Application Laid-Open No. 5-95946 discloses a method of local sound velocity value measurement as described below. In the method of Japanese Patent Application Laid-Open No. 5-95946, ultrasound waves are transmitted from a wave-transmitting transducer into an object while varying an output angle of the waves. The ultrasound waves are received by a wave-receiving transducer while varying an incidence angle of the waves, and all amounts of time elapsed from wave transmission to wave reception are stored in a memory. Next, a virtual sound-velocity distribution is set and, on the basis of the sound-velocity distribution, amounts of elapsed time are calculated for each output angle and each incidence angle. Then, the virtual sound-velocity distribution is corrected, so that a difference between the calculated value of elapsed time and an actual measurement value is minimum, and a sound velocity value within the object is evaluated using a finally obtained sound-velocity distribution.

SUMMARY OF THE INVENTION

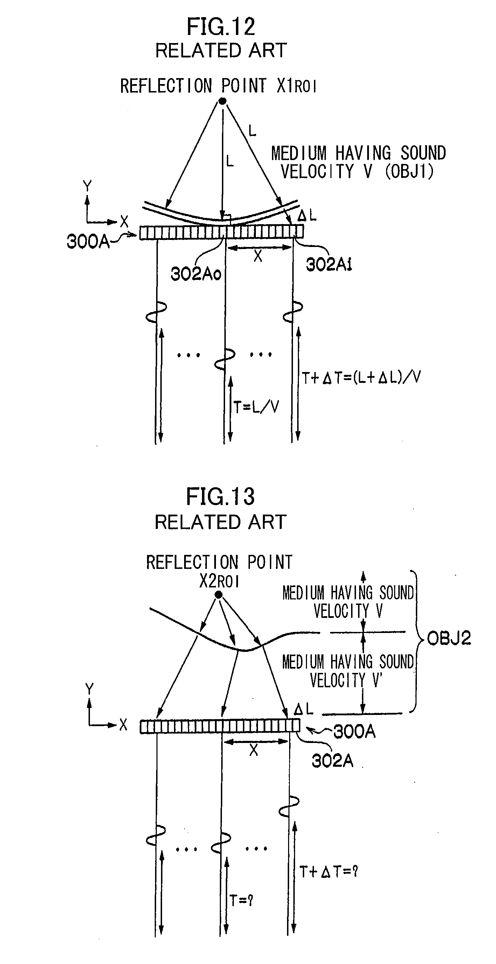

A sound velocity value V within an object OBJ1 formed of a medium having a constant sound velocity value can be calculated as described below. As illustrated in Fig. 12, if a distance from a reflection point (region) $X1_{ROI}$ within the object OBJ1 to an ultrasound probe 300A is L, then an elapsed time T from when ultrasound waves are reflected at the reflection point $X1_{ROI}$ to when the ultrasound waves are received by an element 302A0 immediately below the reflection point $X1_{ROI}$, is T = L/V. If the amount of time that has elapsed until the ultrasound waves are received by an element 302Ai in a position at a distance X from the element 302A0 in an X direction (array direction of elements 302A) is T + $\Delta$T, then a delay time $\Delta$T between the elements 302A0 and 302Ai is represented by the following equation (1).

$$\Delta T = \Delta L / V \ (\text{wherein, } \Delta L = \sqrt{L^2 + X^2} - L) \quad ... (1)$$

Consequently, by measuring the amounts of elapsed time [2T, 2T + $\Delta$T] from when ultrasound waves are transmitted and reflected at the reflection point $X1_{ROI}$ a time T later to when the ultrasound waves are received by each element, it is possible to uniquely determine a distance L to the reflection point $X1_{ROI}$ and a velocity V.

Note that if ultrasound waves received from the reflection point $X1_{ROI}$ can be identified distinctly, it is possible to determine L and V from an elapsed time measured in two different elements whose positional relationship is known.

In general, however, an ultrasound detection signal output from each element 302A is the result of signals from an large number of reflection points having interfered with one another. Thus, it is difficult to discriminate only the signal from a specific reflection point.

Accordingly, a distance L to the reflection point $X1_{ROI}$, a delay time $\Delta$T and a sound velocity value V are, in practice, uniquely determined from the spatial frequency, sharpness and contrast of a reconstructed image in a region of interest near the reflection point $X1_{ROI}$.

If a sound velocity within an object is constant, as described above, it is possible to determine a sound velocity value. If the sound velocity within the object is not constant, however, as in an object OBJ2 illustrated in Fig. 13, it is difficult for the above-described method to determine the distance L to a reflection point (region) $X2_{ROI}$ and sound velocity values V and V'.

The presently disclosed subject matter has been accomplished in view of such circumstances as described above. An object of the presently disclosed subject matter therefore is to provide an ultrasound diagnostic apparatus and the signal processing method thereof capable of precisely calculating a sound velocity value (local sound velocity value) in an arbitrary site under diagnosis within an object.

In order to achieve the aforementioned object, an ultrasound diagnostic apparatus according to a first aspect of the presently disclosed subject matter is configured to include: an ultrasound probe including a plurality of ultrasound transducers for transmitting ultrasound waves to an object and receiving ultrasound wave echoes reflected by the object to output an ultrasound detection signal; a region-of-interest setting device which sets a region of interest within the object; a grid point setting device which sets a grid point to be measured within the region of interest; an ambient sound velocity calculation device which calculates the ambient sound velocity of the region of interest by the transmission/reception of the ultrasound waves with the ultrasound probe; an ambient sound velocity comparison device which compares an ambient sound-velocity distribution for each plurality of assumed local sound velocities with the ambient sound velocity of the region of interest calculated by the ambient sound velocity calculation device; and a local sound velocity decision device which decides a local sound velocity at the grid point to be measured on the basis of the ambient sound velocity of the region of interest.

In the ultrasound diagnostic apparatus according to the first aspect, the grid point setting device sets a grid point to be measured within the region of interest set by the region-of-interest setting device; the ambient sound velocity calculation device calculates the ambient sound velocity of the region of interest by the transmission/reception of the ultrasound waves with the ultrasound probe, the ambient sound velocity comparison device compares an ambient sound-velocity distribution for each plurality of assumed local sound velocities with the ambient sound velocity of the region of interest calculated by the ambient sound velocity calculation device, and the local sound velocity decision device decides a local sound velocity at the grid point to be measured on the basis of the ambient sound velocity of the region of interest, thereby enabling the precise calculation of a sound velocity value (local sound velocity value) in an arbitrary site under diagnosis within an object.

According to a second aspect of the presently disclosed subject matter, in the ultrasound diagnostic apparatus according to the first aspect, preferably, the local sound velocity decision device decides an assumed local sound velocity of the ambient sound-velocity distribution having the smallest error from the ambient sound velocity of the region of interest calculated by the ambient sound velocity calculation device as a local sound velocity at the grid point to be measured.

According to a third aspect of the presently disclosed subject matter, in the ultrasound diagnostic apparatus according to the first or second aspect, preferably, the ambient sound-velocity distribution is an ambient sound-velocity distribution for each plurality of assumed local sound velocities in a biological model previously calculated and stored in a storage device.

According to a fourth aspect of the presently disclosed subject matter, in the ultrasound diagnostic apparatus according to the third aspect, preferably, the ambient sound-velocity distribution is a one-dimensional ambient sound-velocity distribution in a depth direction of the object.

According to a fifth aspect of the presently disclosed subject matter, in the ultrasound diagnostic apparatus according to the third aspect, preferably, the ambient sound-velocity distribution is a two-dimensional ambient sound-velocity distribution in a depth direction of the object.

According to a sixth aspect of the presently disclosed subject matter, the ultrasound diagnostic apparatus according to the first or second aspect preferably further includes a virtual grid point setting device which sets a plurality of virtual grid points in a region shallower than the grid point to be measured on the basis of the ultrasound detection signal, wherein the ambient sound velocity comparison device includes a first calculation device which calculates, on the basis of the ambient sound velocity in the region of interest, a first receiving wave received from the region of interest when the ultrasound waves are transmitted to the region of interest, and a second calculation device which determines, on the basis of the assumed local sound velocity and the ambient sound velocity in the region of interest, a plurality of second receiving waves received from each of the virtual grid points when the ultrasound waves are transmitted to the region of interest, assuming the assumed local sound velocity at the grid point to be measured, and obtains a synthesized receiving wave by synthesizing the plurality of second receiving waves, thereby calculating the ambient sound-velocity distribution for each of the assumed local sound velocity on the basis of the first receiving wave and the synthesized receiving wave.

According to a seventh aspect of the presently disclosed subject matter, in the ultrasound diagnostic apparatus according to the sixth aspect, preferably, the grid point setting device sets a plurality of the grid points to be measured in a region deeper than the plurality of virtual grid points.

According to an eighth aspect of the presently disclosed subject matter, in the ultrasound diagnostic apparatus according to the seventh aspect, preferably, the grid point setting device one-dimensionally sets a plurality of the grid points to be measured in a depth direction.

According to a ninth aspect of the presently disclosed subject matter, in the ultrasound diagnostic apparatus according to the seventh aspect, preferably, the grid point setting device two-dimensionally sets a plurality of the grid points to be measured in a depth direction.

A signal processing method of an ultrasound diagnostic apparatus according to a tenth aspect of the presently disclosed subject matter includes: a region-of-interest setting step of setting a region of interest within the object; a grid point setting step of setting a grid point to be measured within the region of interest; an ambient sound velocity calculation step of calculating the ambient sound velocity of the region of interest by the transmission/reception of the ultrasound waves with an ultrasound probe including a plurality of ultrasound transducers for transmitting ultrasound waves to an object and receiving ultrasound echoes reflected by the object to output an ultrasound detection signal; an ambient sound velocity comparison step of comparing an ambient sound-velocity distribution for each plurality of assumed local sound velocities with the ambient sound velocity of the region of interest calculated by the ambient sound velocity calculation step; and a local sound velocity decision step of deciding a local sound velocity at the grid point to be measured on the basis of the ambient sound velocity of the region of interest.

According to an eleventh aspect of the presently disclosed subject matter, in the signal processing method of an ultrasound diagnostic apparatus according to the tenth aspect, preferably, the local sound velocity decision step includes deciding an assumed local sound velocity of the ambient sound-velocity distribution having the smallest error from the ambient sound velocity of the region of interest calculated by the ambient sound velocity calculation step as a local sound velocity at the grid point to be measured.

According to a twelfth aspect of the presently disclosed subject matter, in the signal processing method of an ultrasound diagnostic apparatus according to the tenth or eleventh aspect, preferably, the ambient sound-velocity distribution is an ambient sound-velocity distribution for each plurality of assumed local sound velocities in a biological model previously calculated and stored in a storage device.

According to a thirteenth aspect of the presently disclosed subject matter, in the signal processing method of an ultrasound diagnostic apparatus according to the twelfth aspect, preferably, the ambient sound-velocity distribution is a one-dimensional ambient sound-velocity distribution in the depth direction of the object.

According to a fourteenth aspect of the presently disclosed subject matter, in the signal processing method of an ultrasound diagnostic apparatus according to the twelfth aspect, preferably, the ambient sound-velocity distribution is a two-dimensional ambient sound-velocity distribution in a depth direction of the object.

According to a fifteenth aspect of the presently disclosed subject matter, the signal processing method of an ultrasound diagnostic apparatus according to the tenth or eleventh aspect preferably further includes a virtual grid point setting step of setting a plurality of virtual grid points in a region shallower than the grid point to be measured on the basis of the ultrasound detection signal, wherein the ambient sound velocity comparison step includes a first calculation step of calculating a first receiving wave received from the region of interest when the ultrasound waves are transmitted to the region of interest on the basis of the ambient sound velocity at the region of interest, and a second calculation step of determining, on the basis of the assumed local sound velocity and the ambient sound velocity in the region of interest, a plurality of second receiving waves received from each of the virtual grid points when the ultrasound waves are transmitted to the region of interest, assuming the assumed local sound velocity at the grid point to be measured, and obtains a synthesized receiving wave by synthesizing the plurality of second receiving waves, thereby calculating the ambient sound-velocity distribution for each of the assumed local sound velocities on the basis of the first receiving wave and the synthesized receiving wave.

According to a sixteenth aspect of the presently disclosed subject matter, in the signal processing method of an ultrasound diagnostic apparatus according to the fifteenth aspect, preferably, the grid point setting step sets a plurality of the grid points to be measured in a region deeper than the plurality of virtual grid points.

According to a seventeenth aspect of the presently disclosed subject matter, in the signal processing method of an ultrasound diagnostic apparatus according to the sixteenth aspect, preferably, the grid point setting step one-dimensionally sets a plurality of the grid points to be measured in a depth direction.

According to an eighteenth aspect of the presently disclosed subject matter, in the signal processing method of an ultrasound diagnostic apparatus according to the sixteenth aspect, preferably, the grid point setting step two-dimensionally sets a plurality of the grid points to be measured in a depth direction.

As has been described heretofore, according to the presently disclosed subject matter, there is provided an advantageous effect of being able to precisely calculate a sound velocity value (local sound velocity value) in an arbitrary site under diagnosis within the object.

BRIEF DESCRIPTION OF THE DRAWINGS

Fig. 1 is a block diagram illustrating an ultrasound diagnostic apparatus according to a first embodiment of the presently disclosed subject matter;

Fig. 2 is a block diagram illustrating the configuration of substantial parts of a data analysis unit of Fig. 1;

Fig. 3 is a flowchart illustrating a flow of processing for calculating the local sound velocity of a region of interest ROI by the data analysis unit of Fig. 2;

Fig. 4 is a schematic view illustrating a region of interest ROI and grid points $X_{ROI}$ set by a region-of-interest and grid point setting unit in the processing of Fig. 3;

Fig. 5 is a graphical view illustrating a one-dimensional ambient sound velocity depth profile for each first assumed local sound velocity used in the processing of Fig. 3;

Fig. 6 is a graphical view illustrating a one-dimensional ambient sound velocity depth profile for each second assumed local sound velocity used in the processing of Fig. 3;

Fig. 7 is a schematic view illustrating a two-dimensional ambient sound velocity depth profile for each assumed local sound velocity used in the processing of Fig. 3;

Fig. 8 is a flowchart illustrating a flow of processing for calculating the local sound velocity of a region of interest ROI by a data analysis unit according to a second embodiment of the presently disclosed subject matter;

Fig. 9 is a drawing used to explain the processing of Fig. 8;

Fig. 10 is a flowchart illustrating a flow of processing for calculating the local sound velocity in a region of interest ROI by a data analysis unit according to a third embodiment of the presently disclosed subject matter;

Fig. 11 is a drawing used to explain the processing of Fig. 10;

Fig. 12 is a first drawing schematically illustrating a conventional process of calculating a local sound velocity value; and

Fig. 13 is a second drawing schematically illustrating a conventional process of calculating a local sound velocity value.

## DETAILED DESCRIPTION OF THE PREFERRED EMBODIMENTS

Hereinafter, preferred embodiments of an ultrasound diagnostic apparatus and the signal processing method thereof according to the presently disclosed subject matter will be described with reference to the accompanying drawings.

First Embodiment:

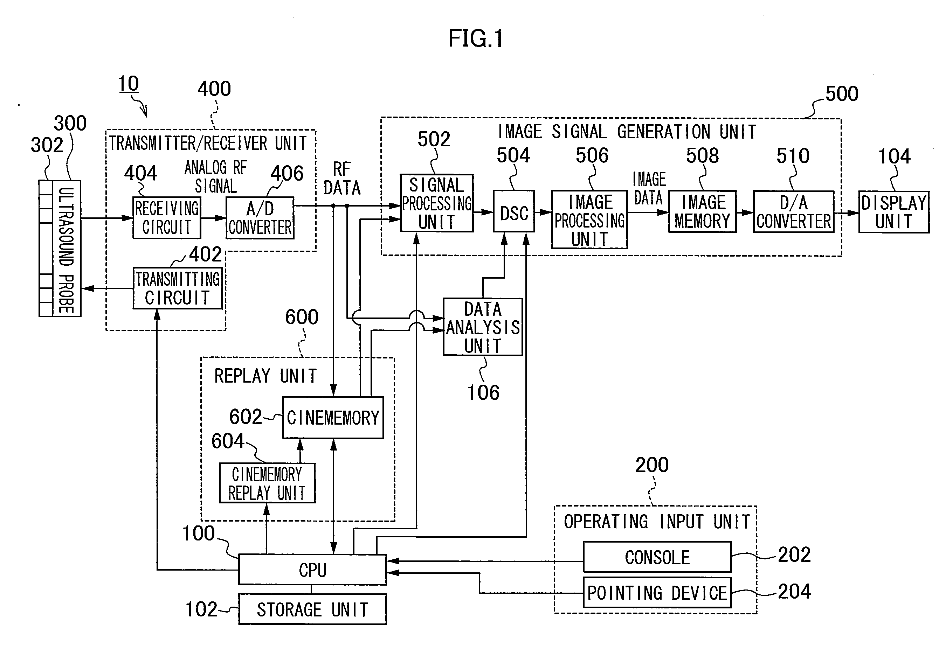

Fig. 1 is a block diagram illustrating an ultrasound diagnostic apparatus according to a first embodiment of the presently disclosed subject matter.

An ultrasound diagnostic apparatus 10 illustrated in Fig. 1 transmits ultrasound beams from an ultrasound probe 300 to an object OBJ, receives ultrasound beams reflected by the object OBJ (hereinafter referred to as ultrasound echoes), and creates and displays an ultrasound image from a detection signal of the ultrasound echoes.

A CPU (Central Processing Unit) 100 controls the respective blocks of the ultrasound diagnostic apparatus 10 according to an operating input from an operating input unit 200.

The operating input unit 200 is an input device adapted to accept an operating input from an operator and includes a console 202 and a pointing device 204. The console 202 includes a keyboard for accepting an input of textual information (for example, patient information), a display mode changeover button for switching a display mode between a mode of independently displaying an amplitude image (B-mode image) and a mode of displaying the result of determining a local sound velocity value, a freeze button for instructing switching between a live mode and a freeze mode, a cinememory replay button for instructing the replay of a cinememory, and an analysis/measurement button for instructing the analysis/measurement of an ultrasound image. The pointing device 204 is used to accept an input for selecting a region on the screen of a display unit 104 and is, for example, a track ball or a mouse. Note that as the pointing device 204, a touch-sensitive panel can also be used.

A storage unit 102 is a memory unit for storing control programs for the CPU 100 to execute the control of the respective blocks of the ultrasound diagnostic apparatus 10 and is, for example, a hard disk or a semiconductor memory.

The display unit 104 is, for example, a CRT (Cathode Ray Tube) display or an LCD (Liquid Crystal Display), and displays ultrasound images (moving and still images) and various setup screens.

The ultrasound probe 300 is a probe abutted on the object OBJ when in use and includes a plurality of ultrasound transducers 302 constituting a one-dimensional or two-dimensional transducer array. Each ultrasound transducer 302 transmits ultrasound beams to the object OBJ on the basis of a drive signal applied from a transmitting circuit 402, and receives ultrasound echoes reflected from the object OBJ to output a detection signal.

Each ultrasound transducer 302 contains an oscillator configured by forming electrodes at both ends of a material having piezoelectricity (piezoelectric substance). As a piezoelectric substance composing the oscillator, piezoelectric ceramic, such as PZT (Pb (lead) zirconate titanate), or a polymer piezoelectric element, such as PVDF (polyvinylidene difluoride), can be used, for example. When an electrical signal is sent to the electrodes of the oscillator to apply a voltage thereto, the piezoelectric substance expands and contracts. The expansion and contraction of this piezoelectric substance generates ultrasound waves in each oscillator. For example, if a pulsing electrical signal is sent to the electrodes of the oscillator, pulsed ultrasound waves are generated and, if a continuous-wave electrical signal is sent to the electrodes of the oscillator, continuous ultrasound waves are generated. Ultrasound waves generated in the respective oscillators are synthesized to form ultrasound beams. In addition, when ultrasound waves are received by each oscillator, the piezoelectric substance of the oscillator expands and contracts, thereby generating an electrical signal. The electrical signal generated in each oscillator is output to a receiving circuit 404 as a detection signal of ultrasound waves.

Note that as the ultrasound transducers 302, it is possible to use elements of plural types different in ultrasound conversion method. For example, an oscillator comprised of the abovementioned piezoelectric substance may be used as an element for transmitting ultrasound waves, and a photodetection method-based ultrasound transducer may be used as an element for receiving ultrasound waves. Here, the photodetection method-based ultrasound transducer is used to detect an ultrasound signal by converting the signal into an optical signal and is, for example, a Fabry-Perot resonator or a fiber Bragg grating.

Next, ultrasound diagnostic processing in a live mode will be described. The live mode is a mode for performing the display, analysis and measurement of an ultrasound image (moving image) obtained by abutting the ultrasound probe 300 on the object OBJ and transmitting and receiving ultrasound waves.

When the ultrasound probe 300 is abutted on the object OBJ and ultrasound diagnosis is initiated by an instruction input from the operating input unit 200, the CPU 100 outputs a control signal to a transmitter/receiver unit 400 to cause the transmitter/receiver unit to begin transmitting ultrasound beams to the object OBJ and receiving ultrasound echoes from the object OBJ. The CPU 100 sets the direction of ultrasound beam transmission and the direction of ultrasound echo reception for each ultrasound transducer 302.

In addition, the CPU 100 selects a transmission delay pattern according to the direction of ultrasound beam transmission and selects a reception delay pattern according to the direction of ultrasound echo reception. Here, transmission delay patterns refer to delay time pattern data given to a drive signal, in order to form ultrasound beams in a desired direction by using ultrasound waves transmitted from the plurality of ultrasound transducers 302. Reception delay patterns refer to delay time pattern data given to a detection signal, in order to extract ultrasound echoes from a desired direction by using ultrasound waves received by the plurality of ultrasound transducers 302. The transmission delay patterns and the reception delay patterns are previously stored in the storage unit 102. The CPU 100 selects a transmission delay pattern and a reception delay pattern from among the transmission delay patterns and reception delay patterns stored in the storage unit 102. According to the selected transmission delay pattern and reception delay pattern, the CPU 100 outputs a control signal to the transmitter/receiver unit 400 and performs the transmission/reception control of ultrasound waves.

The transmitting circuit 402 generates a drive signal according to the control signal from the CPU 100 and applies the drive signal to the ultrasound transducers 302. At this time, the transmitting circuit 402 delays a drive signal to be applied to each ultrasound transducer 302 on the basis of the transmission delay pattern selected by the CPU 100. Here, the transmitting circuit 402 adjusts (delays) a timing to apply a drive signal to each ultrasound transducer 302, so that ultrasound waves transmitted from the plurality of ultrasound transducers 302 form ultrasound beams. Note that the timing to apply a drive signal may be adjusted, so that ultrasound waves transmitted at one time from the plurality of ultrasound transducers 302 reach the entire imaging region of the object OBJ.

The receiving circuit 404 receives and amplifies an ultrasound detection signal output from each ultrasound transducer 302. Since a distance between each ultrasound transducer 302 and an ultrasound reflection source within the object OBJ differs, as described above, the time at which reflected waves arrive at each ultrasound transducer 302 differs. The receiving circuit 404 includes a delay circuit (not illustrated) and delays each detection signal by an amount of time corresponding to a difference in the arrival time of reflected waves (delay time), according to a sound velocity (hereinafter referred to as an assumed sound velocity) or a sound velocity distribution set on the basis of a reception delay pattern selected by the CPU 100. Next, the receiving circuit 404 performs a matching addition on the detection signal to which the delay time has been given, thereby performing reception focusing processing (focusing processing on the reception side). If there are other ultrasound reflection sources in positions different from that of an ultrasound reflection source $X_{ROI}$, ultrasound detection signals from the other ultrasound reflection sources differ in arrival time. Consequently, an addition performed by the addition circuit (not illustrated) of the receiving circuit 404 causes the phases of the ultrasound detection signals from the other ultrasound reflection sources to cancel each other. This causes a reception signal from the ultrasound reflection source $X_{ROI}$, to be largest, and therefore, the reception signal comes into focus. Thus, a sound ray signal (hereinafter referred to as an RF signal) in which a focus of ultrasound echoes is narrowed is formed by the reception focusing processing.

An A/D (Analog-Digital) converter 406 converts the analog RF signal output from the receiving circuit 404 into a digital RF signal (hereinafter referred to as RF data). Here, the RF data contains the phase information of a receiving wave (carrier wave). The RF data output from the A/D converter 406 is input to a signal processing unit 502 and a cinememory 602, respectively.

The cinememory 602 sequentially stores RF data input from the A/D converter 406. In addition, the cinememory 602 stores frame rate-related information (for example, the depth of a position where ultrasound waves are reflected, the density of scanning lines, and a parameter indicating the width of a visual field) input from the CPU 100 in association with the RF data.

The signal processing unit 502 makes a distance-based attenuation correction to the RF data by means of STC (Sensitivity Time gain Control), according to the depth of reflection positions of ultrasound waves.

After that, the signal processing unit 502 performs envelop detection processing, thereby generating B-mode image data (image data in which the amplitude of ultrasound echoes is represented by the brightness of points (luminance)).

The B-mode image data generated by the signal processing unit 502 is data obtained by a scanning method different from a commonly-used television signal scanning method. Accordingly, the DSC (Digital Scan Converter) 504 coverts (raster-converts) the B-mode image data into common image data (for example, image data for a television signal scanning method (for example, NTSC (National Television System Committee) method). The image processing unit 506 performs various types of necessary image processing (for example, gradation processing) on the image data input from the DSC 504.

An image memory 508 stores image data input from the image processing unit 506. A D/A converter 510 converts image data read out of the image memory 508 into an analog image signal and outputs the image signal to a display unit 104. Consequently, an ultrasound image (moving image) taken by the ultrasound probe 300 is displayed on the display unit 104.

Note that although in the present embodiment, a detection signal subjected to reception focusing processing at the receiving circuit 404 is defined as an RF signal, a detection signal not subjected to reception focusing processing may alternatively be defined as the RF signal. In this case, a plurality of ultrasound detection signals output from a plurality of ultrasound transducers 302 is amplified at the receiving circuit 404, and the amplified detection signals, i.e., RF signals are A/D-converted at the A/D converter 406, thereby generating RF data. Then, the RF data is supplied to the signal processing unit 502 and is stored in the cinememory 602. The reception focusing processing is performed digitally at the signal processing unit 502.

Next, a cinememory replay mode will be described. The cinememory replay mode is a mode for displaying, analyzing and measuring an ultrasound diagnosis image on the basis of RF data stored in the cinememory 602.

When the cinememory replay button of the console 202 is pressed, the CPU 100 switches the operating mode of the ultrasound diagnostic apparatus 10 to the cinememory replay mode. When in the cinememory replay mode, the CPU 100 instructs a cinememory replay unit 604 to replay RF data specified by an operating input from an operator. The cinememory replay unit 604 reads RF data out of the cinememory 602, according to an instruction from the CPU 100, to transmit the RF data to the signal processing unit 502 of an image signal generation unit 500. The RF data transmitted from the cinememory 602 undergoes predetermined processing (the same processing as in live mode) at the signal processing unit 502, the DSC 504, and the image processing unit 506, so as to be converted into image data, and is then output through the image memory 508 and the D/A converter 510 to the display unit 104. Consequently, an ultrasound image (moving image or still image) based on the RF data stored in the cinememory 602 is displayed on the display unit 104.

If the freeze button of the console 202 is pressed while an ultrasound image (moving image) is being displayed in the live mode or the cinememory replay mode, the ultrasound image displayed when the freeze button is pressed is displayed on the display unit 104 as a still image. Consequently, the operator can show and observe a still image of a region of interest (ROI).

When the measurement button of the console 202 is pressed, analysis/measurement specified by an operating input from the operator is performed at the data analysis unit 106.

The data analysis unit 106 acquires RF data before the application of image processing from the A/D converter 406 or the cinememory 602 when the measurement button is pressed in each operating mode. In addition to determining the local sound velocity of the region of interest by using the RF data, the data analysis unit 106 performs various types of analysis/measurement specified by the operator (for example, analysis of strain in tissues (hardness diagnosis), bloodstream measurement, measurement of the movement of tissues, or IMT (Intima-Media Thickness) measurement). The results of analysis/measurement by the data analysis unit 106 are output to the DSC 504 of the image signal generation unit 500. The DSC 504 inserts the results of analysis/measurement by the data analysis unit 106 into the image data of the ultrasound image and outputs the ultrasound image to the display unit 104. Consequently, the ultrasound image and the analysis/measurement results are displayed on the display unit 104.

In addition, when a display mode changeover button is pressed, the display mode switches among a mode of independently displaying a B-mode image, a mode of displaying the results of local sound velocity value determination with superimposed on the B-mode image (for example, a mode of displaying the B-mode image with the image color-coded or with the brightness of the image varied according to the local sound velocity value, or a mode of displaying the B-mode image in which points equal in local sound velocity value are connected with lines), and a mode of displaying the B-mode image and the image of the results of local sound velocity value determination in parallel with each other. Consequently, the operator can, for example, discover lesions by observing the results of local sound velocity value determination. Regions of interest may be set by the operator or may be successively set in predetermined size in the order of the shallowest to deepest regions.

Note that a B-mode image obtained by performing at least one of transmission focusing processing (focusing on the transmission side) and reception focusing processing on the basis of the results of local sound velocity value determination may be displayed on the display unit 104.

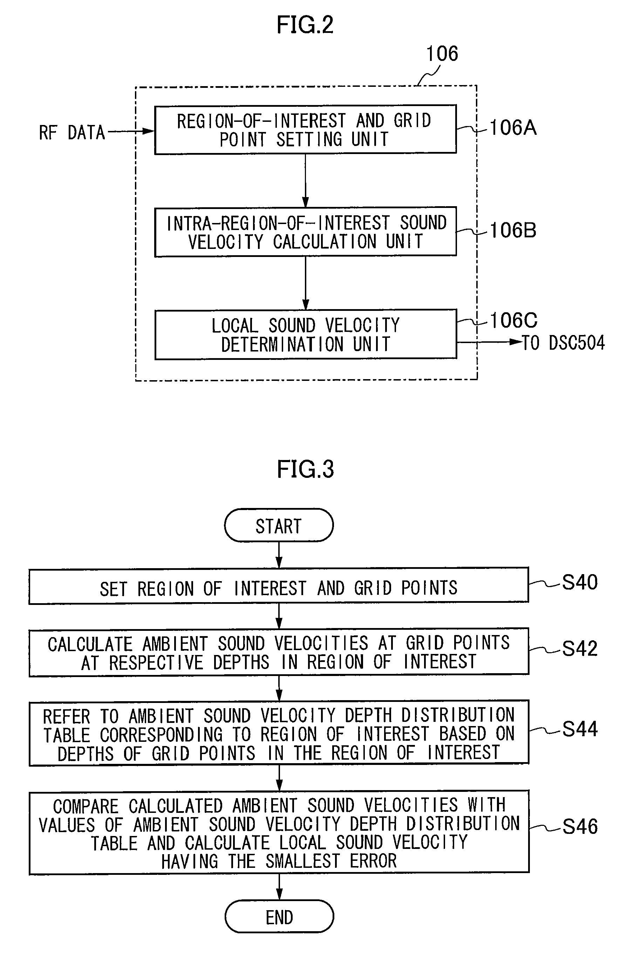

Fig. 2 is a block diagram illustrating the configuration of substantial parts of the data analysis unit of Fig. 1. As illustrated in Fig. 2, the data analysis unit 106 includes a region-of-interest and grid point setting unit 106a, an intra-region-of-interest sound velocity calculation unit 106b, and a local sound velocity determination unit 106c, in order to determine the local sound velocity of a region of interest by using RF data.

The region-of-interest and grid point setting unit 106a sets a region of interest ROI within an object OBJ, and sets grid points $X_{ROI}$ within the region of interest ROI in the depth direction thereof as a plurality of grid points to be measured, and constitutes a region-of-interest setting device and a grid point setting device.

The intra-region-of-interest sound velocity calculation unit 106b is configured to calculate ambient sound velocities at the grid points $X_{ROI}$ at respective depths in the region of interest ROI, and constitutes an ambient sound velocity calculation device.

The local sound velocity determination unit 106c refers to an ambient sound-velocity distribution table for each plurality of assumed sound velocities previously stored in a storage unit (not illustrated) within the unit, compares the values of this ambient sound-velocity distribution table with the values of the ambient sound velocities calculated by the intra-region-of-interest sound velocity calculation unit 106b, extracts an ambient sound-velocity distribution table for assumed local sound velocities having the smallest error, and determines the assumed local sound velocities of the extracted ambient sound-velocity distribution table as local sound velocities in the region of interest ROI. The local sound velocity determination unit 106c constitutes an ambient sound velocity comparison device and a local sound velocity decision device.

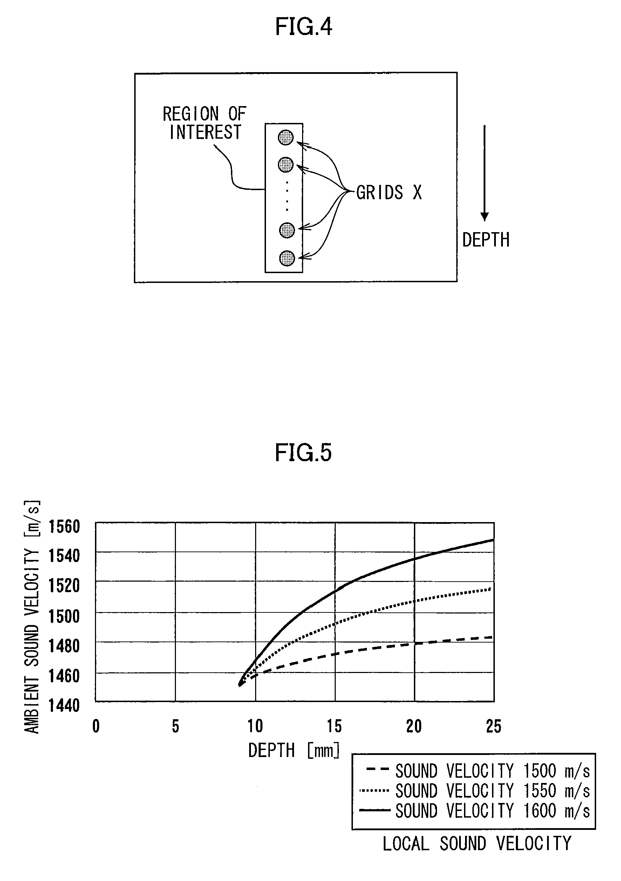

A process of calculating the local sound velocity of the region of interest ROI in the present embodiment configured in this way will be described using the flowchart of Fig. 3. Fig. 3 is a flowchart illustrating a flow of processing for calculating the local sound velocity of the region of interest ROI by the data analysis unit of Fig. 2, whereas Figs. 4 to 6 are drawings used to explain the processing of Fig. 3.

As illustrated in Fig. 3, the data analysis unit 106 sets the region of interest ROI within the object OBJ by the region-of-interest and grid point setting unit 106a, and sets grid points $X_{ROI}$ as a plurality of grid points to be measured within the region of interest ROI in the depth direction thereof (step S40). Fig. 4 illustrates the region of interest ROI and the grid points $X_{ROI}$ on sound rays set by the region-of-interest and grid point setting unit 106a in step S40.

Next, the data analysis unit 106 calculates ambient sound velocities at the grid points $X_{ROI}$ at respective depths in the region of interest ROI by the intra-region-of-interest sound velocity calculation unit 106b (step S42).

Specifically, the data analysis unit 106 determines ambient sound velocity values at all of the grid points $X_{ROI}$ previously set within the object OBJ. Here, an ambient sound velocity value refers to a sound velocity value at which the contrast and sharpness of an image are highest, and does not necessarily correspond to an actual local sound velocity value at each grid point $X_{ROI}$. As a method for determining ambient sound velocity values, it is possible to apply a method of determination from, for example, an image contrast, a spatial frequency in a scan direction, or a variance (see, for example, Japanese Patent Application Laid-Open No. 8-317926).

Then, the data analysis unit 106 refers to ambient sound-velocity distribution tables for each plurality of assumed local sound velocities previously stored by the local sound velocity determination unit 106c (step S44), compares the values of these ambient sound-velocity distribution tables with the values of ambient sound velocities calculated at the intra-region-of-interest sound velocity calculation unit 106b, extracts an ambient sound-velocity distribution table for an assumed local sound velocity having the smallest error, and determines the assumed local sound velocity of the extracted ambient sound-velocity distribution table as a local sound velocity in the region of interest ROI (step S46). Methods for calculating an error from table values include calculating the absolute values of ambient sound velocity value errors at respective depths and calculating an integration value of square values in the depth direction.

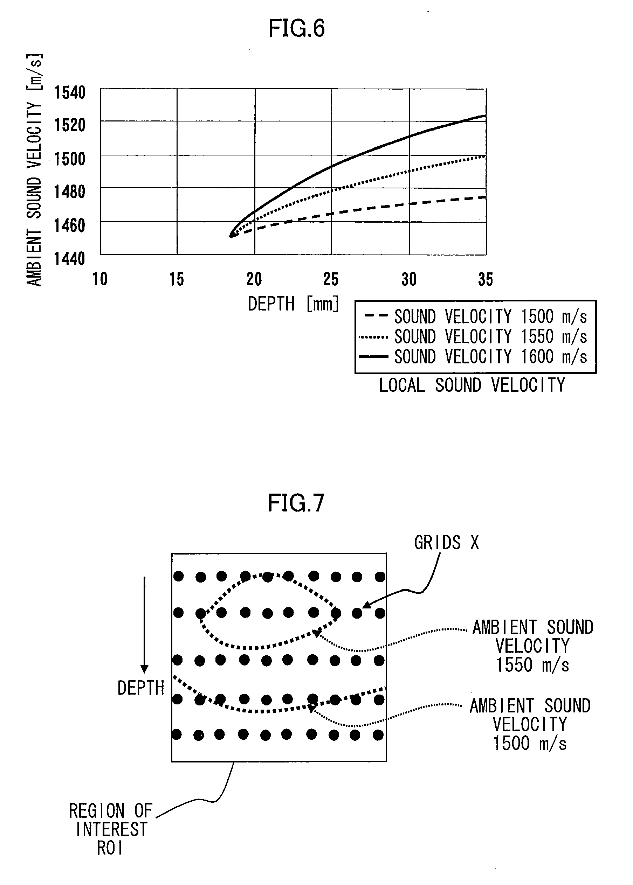

Fig. 5 illustrates an on-the-sound ray (one-dimensional) ambient sound velocity depth profile for each assumed local sound velocity in a biological model in which an ambient sound velocity is 1450 m/sec at a depth shallower than 10 mm. Likewise, Fig. 6 illustrates an on-the-sound ray (one-dimensional) ambient sound velocity depth profile for each assumed local sound velocity in a biological model in which an ambient sound velocity is 1450 m/sec at a depth shallower than 20 mm. As illustrated in Figs. 5 and 6, if an ambient sound velocity at the undersurface of a region of interest is given, then an ambient sound velocity depth profile in a region of interest deeper than the depth of that region is uniquely determined based on an assumed local sound velocity. Consequently, in step S46, the local sound velocity determination unit 106c can determine the local sound velocity of the region of interest ROI (including grid points $X_{ROI}$) from the ambient sound velocity depth profile (where, the undersurface of the region of interest at which the assumed local sound velocity is regarded as constant is assumed to be a planar surface).

As described above, in the present embodiment, an ambient sound velocity is calculated using an amplitude image or RF data obtained when a B-mode image is generated, or data on receiving waves received by each ultrasound transducer element. By comparing this ambient sound velocity with a previously stored ambient sound velocity depth profile for each assumed local sound velocity, it is possible to promptly and precisely determine a local sound velocity value within an object. By using the local sound velocity value thus determined, it is possible to even more precisely detect lesions within the object.

Note that although a description has been given that, in the present embodiment, an ambient sound velocity depth profile for each assumed local sound velocity is previously stored, the ambient sound velocity depth profile may be determined each time by means of model calculation on the basis of the ambient sound velocity at the undersurface of the region of interest and the assumed local sound velocity in the region of interest.

Also note that although a description has been given that, in the present embodiment, the region-of-interest and grid point setting unit 106a one-dimensionally sets a plurality of grid points $X_{ROI}$ in the region of interest ROI on a sound ray (see Fig. 4), the embodiment is not limited to this. Alternatively, as illustrated in Fig. 7, a plurality of grid points $X_{ROI}$ may be two-dimensionally set in the region of interest ROI, and the local sound velocity value within the object may be determined by referring to a two-dimensional ambient sound velocity depth profile for each assumed local sound velocity. By way of example, Fig. 7 illustrates two-dimensional ambient sound velocity depth profiles for 1500 m/sec and 1550 m/sec.

In addition, the present embodiment does not require any configuration dedicated to transmit and receive ultrasound waves in order to measure local sound velocity values.

Second Embodiment:

Next, a second embodiment of the presently disclosed subject matter will be described. The second embodiment is the same in configuration as the first embodiment and differs therefrom only in processing at the data analysis unit 106. Therefore, only the difference will be described.

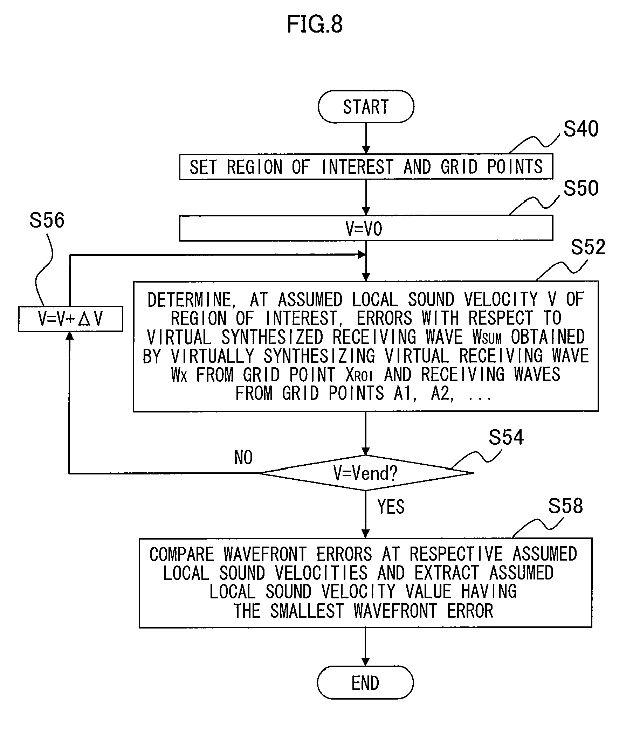

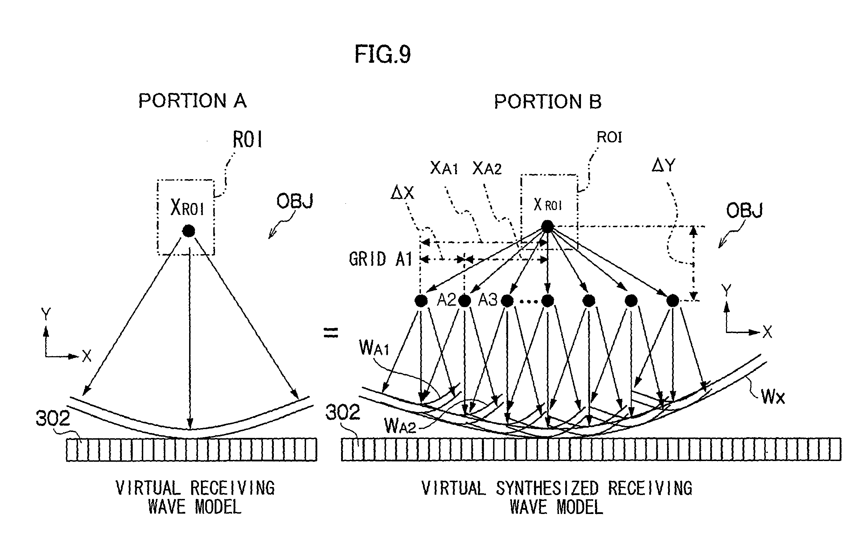

Fig. 8 is a flowchart illustrating a flow of processing for calculating the local sound velocity of a region of interest ROI by a data analysis unit of the second embodiment. Fig. 9 is a drawing used to explain the processing of Fig. 8.

As illustrated in Fig. 8, a data analysis unit 106 sets, by a region-of-interest and grid point setting unit 106a, a plurality of virtual grid points A1, A2, ..., in addition to the region of interest ROI and grid points $X_{ROI}$ as grid points to be measured, in positions shallower than the grid points $X_{ROI}$ (step S40).

Specifically, as illustrated in the virtual synthesized receiving wave model in Portion A of Fig. 9, a grid point representative of a region of interest ROI within an object OBJ is assumed to be $X_{ROI}$, grid points which are located shallower than the grid point $X_{ROI}$ (i.e., closer to ultrasound transducers 302) and arranged at equal spacing (equal interval) in X direction are assumed to be A1, A2, ... , and at least a sound velocity between the grid point $X_{ROI}$ and each of the grid points A1, A2, ... , is assumed to be constant.

In the present embodiment, a local sound velocity value at the grid point $X_{ROI}$ is determined from a positional relationship between the grid point $X_{ROI}$ and each of the grid points A1, A2, ... by assuming that the amounts of elapsed time (T and delay time $\Delta T$) of receiving waves (respectively represented as $W_{A1}$, $W_{A2}$, ... ) from the grid points A1, A2, ... are known. Specifically, advantage is taken of the fact that a virtual receiving wave $W_X$ received from the grid point $X_{ROI}$, as shown in the virtual receiving wave model of Fig. 9 and a virtual synthesized receiving wave $W_{SUM}$ obtained by virtually synthesizing receiving waves received from the grid points A1, A2, ... as shown in the virtual synthesized receiving wave model of Fig. 9 agree with each other ($W_X = W_{SUM}$) according to Huygens' principle.

Here, the range and the number of the grid points A1, A2, ... used in calculations when determining the local sound velocity value at the grid point $X_{ROI}$ are previously decided. If the range of grid points to be used in the calculation of the local sound velocity value is too wide, the error of the local sound velocity value becomes large and, if the range is too narrow, the error of the local sound velocity value from a virtual receiving wave becomes large. Accordingly, the range of grid points is decided based on this trade-off.

The spacing between the respective grid points A1, A2, ... in the X direction is decided based on a trade-off between resolution and processing time. A spacing $\Delta X$ between each two of the respective grid points A1, A2, .., in the X direction is, for example, 1 mm to 1 cm.

If a spacing between the grid point $X_{ROI}$ and each of the grid points A1, A2, ... in a Y direction is too narrow, an error in error calculations becomes large and, if the spacing is too wide, the error of the local sound velocity value becomes large. A spacing $\Delta Y$ between the grid point $X_{ROI}$ and each of the grid points A1, A2, ... in the Y direction is decided on the basis of the image resolution setting of ultrasound images. The spacing $\Delta Y$ between the grid point $X_{ROI}$ and each of the grid points A1, A2, ... in the Y direction is, for example, 1 cm.

After the grid point $X_{ROI}$ and the grid points A1, A2, .., are set, the data analysis unit 106 sets an assumed local sound velocity value V, which is a parameter, to an initial value V0 (step S50).

Then, the data analysis unit 106 calculates, by an intra-region-of-interest sound velocity calculation unit 106b, ambient sound velocities at all of the grid point $X_{ROI}$ and the grid points A1, A2, .., in the region of interest ROI at the assumed local sound velocity value V. In addition, the data analysis unit 106 determines, by the intra-region-of-interest sound velocity calculation unit 106b, the errors of the ambient sound velocities with respect to a virtual synthesized receiving wave $W_{SUM}$ obtained by virtually synthesizing a virtual receiving wave $W_X$ from the grid point $X_{ROI}$, and receiving waves from the grid points A1, A2, ... (step S52).

Subsequently, the data analysis unit 106 determines, by step S52, whether or not the assumed local sound velocity value V, which is a parameter, has reached a final value Vend (V = Vend). If V < Vend, then the data analysis unit 106 adds a predetermined step increment ∆V to V in step S56 and repeats step S52. If V = Vend, then the data analysis unit 106 advances to step S54.

Next, the data analysis unit 106 compares, by a local sound velocity determination unit 106c, wavefront errors (error between the virtual receiving wave $W_X$ and the virtual synthesized receiving wave $W_{SUM}$) at the respective assumed local sound velocities. Then, the data analysis unit 106 extracts an assumed local sound velocity value having the smallest wavefront error, and determines the extracted assumed local sound velocity as a local sound velocity in the region of interest ROI (step S58).

As described above, according to the present embodiment, it is possible to determine the local sound velocity of the region of interest ROI by means of model calculation.

Third Embodiment:

Next, a third embodiment of the presently disclosed subject matter will be described. The present embodiment differs from the second embodiment in processing at the data analysis unit 106. Therefore, only the difference will be described.

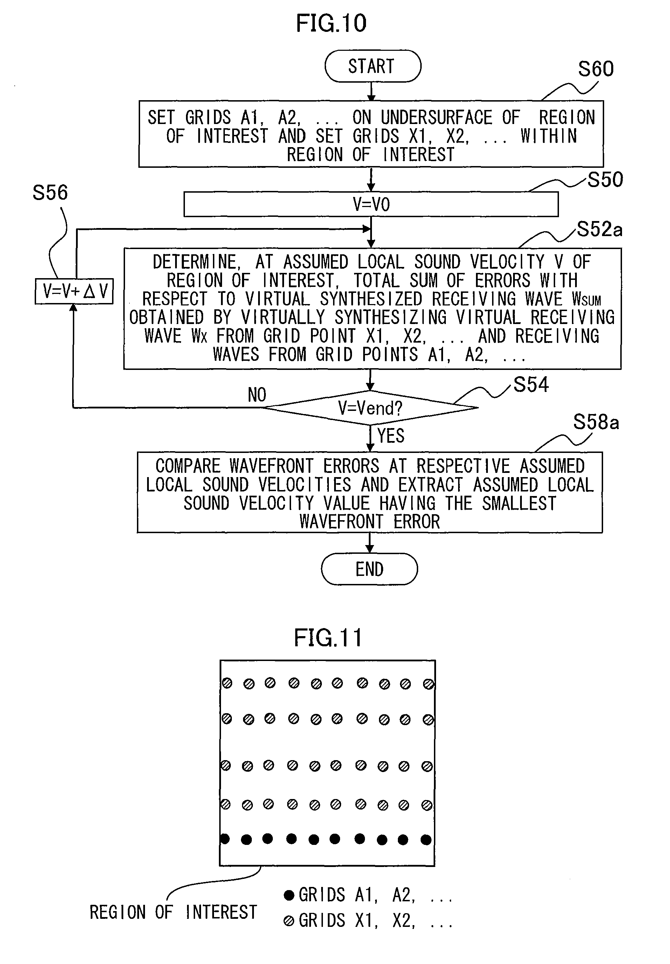

Fig. 10 is a flowchart illustrating a flow of processing for calculating the local sound velocity of a region of interest ROI by a data analysis unit according to the third embodiment of the presently disclosed subject matter. Fig. 11 is a drawing used to explain the processing of Fig. 10.

As illustrated in Fig. 10, the data analysis unit 106 sets, by a region-of-interest and grid point setting unit 106a, a region of interest ROI and virtual grid points A1, A2, ... In addition, as illustrated in Fig. 11, the data analysis unit 106 sets a plurality of grid points X1, X2, ... as grid points to be measured in positions deeper than the virtual grid points A1, A2, ... (step S60).

Then, after the processing of step S50, the data analysis unit 106 calculates, by an intra-region-of-interest sound velocity calculation unit 106b, ambient sound velocities at all of the grid points X1, X2, ... and the grid point A1, A2, ... in the region of interest ROI at an assumed local sound velocity value V. On the basis of the ambient sound velocities, the data analysis unit 106 determines, by an intra-region-of-interest sound velocity calculation unit 106b, the errors of the ambient sound velocities with respect to respective virtual synthesized receiving waves W1, W2, ... obtained by virtually synthesizing respective virtual receiving waves $W_{X1}$, $W_{X2}$, ... from the grid point X1, X2, ... and receiving waves from the grid points A1, A2, ... , thereby calculating a total sum of these errors ($\Sigma$ |Wj - $W_{Xj}$|) (step S52a).

Then, after the processing of steps S54 and S56, the data analysis unit 106 compares, by a local sound velocity determination unit 106c, the total sums of wavefront errors ($\Sigma$ |Wj - $W_{Xj}$|) at respective assumed local sound velocities. Subsequently, the data analysis unit 106 extracts an assumed local sound velocity value having the smallest error, and determines the extracted assumed local sound velocity as a local sound velocity in the region of interest ROI (step S58a).

As described above, according to the present embodiment, a local sound velocity is decided on the basis of a total sum of the wavefront errors of a plurality of the grid points X1, X2, ... within the region of interest, whereas in the second embodiment, the local sound velocity is decided on the basis of a wavefront error of only the grid point $X_{ROI}$ representative of the region of interest. Consequently, it is possible to precisely decide the local sound velocity.

Here, by setting the grid points A1, A2, ... on a curved surface rather than on a planar surface, it is possible to decide the local sound velocity of the region of interest in consideration of the two-dimensional shape thereof. Consequently, it is possible to decide the local sound velocity more precisely, compared with the first embodiment. As a method for setting the grid points on the curved surface, a user may set the grid points or the grid points may be set by means of edge detection processing or high-brightness portion detection processing. In addition, templates for the undersurface shapes of the region of interest, and corresponding ambient sound-velocity distributions may be retained previously. Then, errors in all of the shape templates and local sound velocities may be calculated by comparing the local sound velocities with the previously retained ambient sound-velocity distributions instead of making model calculations. Then, a shape and a local sound velocity value having the smallest error may be determined.

Note that in the first embodiment, wavefront errors may be used in place of ambient sound-velocity distribution errors. Also note that in the second and third embodiments, ambient sound-velocity distribution errors may be used in place of wavefront errors.

While an ultrasound diagnostic apparatus and the signal processing method thereof of the presently disclosed subject matter have been heretofore described in detail, the presently disclosed subject matter is not limited to the above-described embodiments. Accordingly, it is needless to say that various improvements and modifications may be applied to the presently disclosed subject matter without departing from the subject matter thereof.

**Claims**

1. An ultrasound diagnostic apparatus (10) comprising:

   an ultrasound probe (300) including a plurality of ultrasound transducers (302) for transmitting ultrasound waves to an object and receiving ultrasound echoes reflected by the object to output an ultrasound detection signal;
   a region-of-interest setting device (106A) which sets a region of interest within the object;
   a grid point setting device (106A) which sets a grid point (X) to be measured within the region of interest;
   an ambient sound velocity calculation device (106B) which calculates the ambient sound velocity of the region of interest by the transmission/reception of the ultrasound waves with the ultrasound probe (300);
   an ambient sound velocity comparison device (106C) which compares an ambient sound-velocity distribution for each plurality of assumed local sound velocities with the ambient sound velocity of the region of interest calculated by the ambient sound velocity calculation device (106B); and
   a local sound velocity decision device (106C) which decides a local sound velocity at the grid point (X) to be measured on the basis of the ambient sound velocity of the region of interest.

2. The ultrasound diagnostic apparatus (10) according to claim 1, wherein the local sound velocity decision device (106C) decides an assumed local sound velocity of the ambient sound-velocity distribution having the smallest error from the ambient sound velocity of the region of interest calculated by the ambient sound velocity calculation device (106B) as a local sound velocity at the grid point (X) to be measured.

3. The ultrasound diagnostic apparatus (10) according to claim 1 or 2, wherein the ambient sound-velocity distribution is an ambient sound-velocity distribution for each plurality of assumed local sound velocities in a biological model previously calculated and stored in a storage device (102).

4. The ultrasound diagnostic apparatus (10) according to claim 3, wherein the ambient sound-velocity distribution is a one-dimensional ambient sound-velocity distribution in a depth direction of the object.

5. The ultrasound diagnostic apparatus (10) according to claim 3, wherein the ambient sound-velocity distribution is a two-dimensional ambient sound-velocity distribution in a depth direction of the object.

6. The ultrasound diagnostic apparatus (10) according to claim 1 or 2, further comprising
   a virtual grid point setting device (106A) which sets a plurality of virtual grid points (A1, A2, ...) in a region shallower than the grid point (X) to be measured on the basis of the ultrasound detection signal, wherein
   the ambient sound velocity comparison device (106C) includes:

   a first calculation device which calculates, on the basis of the ambient sound velocity in the region of interest, a first receiving wave received from the region of interest when the ultrasound waves are transmitted to the region of interest; and
   a second calculation device which determines, on the basis of the assumed local sound velocity and the ambient sound velocity in the region of interest, a plurality of second receiving waves received from each of the virtual grid points (A1, A2, ...) when the ultrasound waves are transmitted to the region of interest, assuming the assumed local sound velocity at the grid point (X) to be measured, and obtains a synthesized receiving wave by synthesizing the plurality of second receiving waves,
   and calculates the ambient sound-velocity distribution for each of the assumed local sound velocity on the basis of the first receiving wave and the synthesized receiving wave.

7. The ultrasound diagnostic apparatus (10) according to claim 6, wherein the grid point setting device (106A) sets a plurality of the grid points (X1, X2, ...) to be measured in a region deeper than the plurality of virtual grid points (A1, A2, ...).

8. The ultrasound diagnostic apparatus according to claim 7, wherein the grid point setting device (106A) one-dimensionally sets a plurality of the grid points (X1, X2, ...) to be measured in a depth direction.

9. The ultrasound diagnostic apparatus (10) according to claim 7, wherein the grid point setting device (106A) two-dimensionally sets a plurality of the grid points (X1, X2, ...) to be measured in a depth direction.

10. A signal processing method of an ultrasound diagnostic apparatus, comprising:

    a region-of-interest setting step (S40) of setting a region of interest within an object;
    a grid point setting step (S40) of setting a grid point to be measured within the region of interest;
    an ambient sound velocity calculation step (S42) of calculating the ambient sound velocity of the region of interest by the transmission/reception of the ultrasound waves with an ultrasound probe (300) including a plurality of ultrasound transducers (302) for transmitting ultrasound waves to the object and receiving ultrasound echoes reflected by the object to output an ultrasound detection signal;
    an ambient sound velocity comparison step (S44) of comparing an ambient sound-velocity distribution for each plurality of assumed local sound velocities with the ambient sound velocity of the region of interest calculated by the ambient sound velocity calculation step (S42); and
    a local sound velocity decision step (S46) of deciding a local sound velocity at the grid point to be measured on the basis of the ambient sound velocity of the region of interest.

11. The signal processing method of an ultrasound diagnostic apparatus according to claim 10, wherein the local sound velocity decision step (S46) includes deciding an assumed local sound velocity of the ambient sound-velocity distribution having the smallest error from the ambient sound velocity of the region of interest calculated in the ambient sound velocity calculation step (S44) as a local sound velocity at the grid point to be measured.

12. The signal processing method of an ultrasound diagnostic apparatus according to claim 10 or 11, wherein the ambient sound-velocity distribution is an ambient sound-velocity distribution for each plurality of assumed local sound velocities in a biological model previously calculated and stored in a storage device (102).

13. The signal processing method of an ultrasound diagnostic apparatus according to claim 12, wherein the ambient sound-velocity distribution is a one-dimensional ambient sound-velocity distribution in a depth direction of the object.

14. The signal processing method of an ultrasound diagnostic apparatus according to claim 12, wherein the ambient sound-velocity distribution is a two-dimensional ambient sound-velocity distribution in a depth direction of the object.

15. The signal processing method of an ultrasound diagnostic apparatus according to claim 10 or 11, further comprising a virtual grid point setting step (S40) of setting a plurality of virtual grid points (A1, A2, ...) in a region shallower than the grid point to be measured on the basis of the ultrasound detection signal, wherein the ambient sound velocity comparison step (S44) includes:

    a first calculation step (S50, S52) of calculating a first receiving wave received from the region of interest when the ultrasound waves are transmitted to the region of interest on the basis of the ambient sound velocity at the region of interest; and
    a second calculation step (S54, S56) of determining, on the basis of the assumed local sound velocity and the ambient sound velocity in the region of interest, a plurality of second receiving waves received from each of the virtual grid points (A1, A2, ...) when the ultrasound waves are transmitted to the region of interest, assuming the assumed local sound velocity at the grid point to be measured, and obtains a synthesized receiving wave by synthesizing the plurality of second receiving waves, and
    the ambient sound-velocity distribution for each of the assumed local sound velocities is calculated on the basis of the first receiving wave and the synthesized receiving wave (S58).

16. The signal processing method of an ultrasound diagnostic apparatus according to claim 15, wherein the grid point setting step includes setting (S60) a plurality of the grid points (X1, X2, ...) to be measured in a region deeper than the plurality of virtual grid points (A1, A2, ...).

17. The signal processing method of an ultrasound diagnostic apparatus according to claim 16, wherein the grid point setting step includes one-dimensionally setting a plurality of the grid points (X1, X2, ...) to be measured in a depth direction.

**18.** The signal processing method of an ultrasound diagnostic apparatus according to claim 16, wherein the grid point setting step includes two-dimensionally setting a plurality of the grid points (X1, X2, ...) to be measured in a depth direction.

# FIG.1

# FIG.2

```
                              ┌──────────────────────────────┐
RF DATA ─────────────────────▶│ REGION-OF-INTEREST AND GRID  │ ─── 106A
                              │     POINT SETTING UNIT        │
                              └──────────────────────────────┘
                                           │
                                           ▼
                              ┌──────────────────────────────┐
                              │ INTRA-REGION-OF-INTEREST SOUND│ ─── 106B
                              │   VELOCITY CALCULATION UNIT    │
                              └──────────────────────────────┘
                                           │
                                           ▼
                              ┌──────────────────────────────┐
                              │    LOCAL SOUND VELOCITY       │ ─── 106C
                              │    DETERMINATION UNIT         │ ──────▶ TO DSC504
                              └──────────────────────────────┘
```

106

# FIG.3

START

SET REGION OF INTEREST AND GRID POINTS — S40

CALCULATE AMBIENT SOUND VELOCITIES AT GRID POINTS AT RESPECTIVE DEPTHS IN REGION OF INTEREST — S42

REFER TO AMBIENT SOUND VELOCITY DEPTH DISTRIBUTION TABLE CORRESPONDING TO REGION OF INTEREST BASED ON DEPTHS OF GRID POINTS IN THE REGION OF INTEREST — S44

COMPARE CALCULATED AMBIENT SOUND VELOCITIES WITH VALUES OF AMBIENT SOUND VELOCITY DEPTH DISTRIBUTION TABLE AND CALCULATE LOCAL SOUND VELOCITY HAVING THE SMALLEST ERROR — S46

END

## FIG.4

REGION OF
INTEREST

GRIDS X

DEPTH

## FIG.5

LOCAL SOUND VELOCITY
- - SOUND VELOCITY 1500 m/s
········ SOUND VELOCITY 1550 m/s
— SOUND VELOCITY 1600 m/s

## FIG.6

## FIG.7

# FIG.8

START

S40

SET REGION OF INTEREST AND GRID POINTS

S50

$V=V0$

S56

$V=V+\Delta V$

S52

DETERMINE, AT ASSUMED LOCAL SOUND VELOCITY V OF REGION OF INTEREST, ERRORS WITH RESPECT TO VIRTUAL SYNTHESIZED RECEIVING WAVE $W_{SUM}$ OBTAINED BY VIRTUALLY SYNTHESIZING VIRTUAL RECEIVING WAVE $W_X$ FROM GRID POINT $X_{ROI}$ AND RECEIVING WAVES FROM GRID POINTS A1, A2, ...

S54

NO        $V=Vend$?

YES        S58

COMPARE WAVEFRONT ERRORS AT RESPECTIVE ASSUMED LOCAL SOUND VELOCITIES AND EXTRACT ASSUMED LOCAL SOUND VELOCITY VALUE HAVING THE SMALLEST WAVEFRONT ERROR

END

# FIG.9

PORTION A — VIRTUAL RECEIVING WAVE MODEL

PORTION B — VIRTUAL SYNTHESIZED RECEIVING WAVE MODEL

## FIG.10

START — S60

SET GRIDS A1, A2, ... ON UNDERSURFACE OF REGION OF INTEREST AND SET GRIDS X1, X2, ... WITHIN REGION OF INTEREST

S50

V=V0

S56

V=V+ΔV

S52a

DETERMINE, AT ASSUMED LOCAL SOUND VELOCITY V OF REGION OF INTEREST, TOTAL SUM OF ERRORS WITH RESPECT TO VIRTUAL SYNTHESIZED RECEIVING WAVE Wsum OBTAINED BY VIRTUALLY SYNTHESIZING VIRTUAL RECEIVING WAVE Wx FROM GRID POINT X1, X2, ... AND RECEIVING WAVES FROM GRID POINTS A1, A2, ...

S54

NO — V=Vend?

YES

S58a

COMPARE WAVEFRONT ERRORS AT RESPECTIVE ASSUMED LOCAL SOUND VELOCITIES AND EXTRACT ASSUMED LOCAL SOUND VELOCITY VALUE HAVING THE SMALLEST WAVEFRONT ERROR

END

## FIG.11

REGION OF INTEREST

● GRIDS A1, A2, ...
⊘ GRIDS X1, X2, ...

## FIG.12
### RELATED ART

REFLECTION POINT X1ROI

L

L

MEDIUM HAVING SOUND VELOCITY V (OBJ1)

Y

X

$\Delta$L

300A

302Ao

302Ai

X

$T+\Delta T=(L+\Delta L)/V$

$T=L/V$

## FIG.13
### RELATED ART

REFLECTION POINT X2ROI

MEDIUM HAVING SOUND VELOCITY V

MEDIUM HAVING SOUND VELOCITY V'

OBJ2

Y

X

$\Delta$L

300A

302A

X

$T+\Delta T=?$

$T=?$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5095946 A

- JP 8317926 A